# EUROPEAN PATENT APPLICATION

(11) **EP 3 673 736 A1**
(43) Date of publication of application: **01.07.2020**
(21) Application number: 19211027.8
(22) Date of filing: 24.02.2010
(51) Int. Cl.: A01N 43/36, A61K 31/40, A61P 29/00, A61P 27/00, A61K 9/06, A61K 31/79, A61K 9/00, A61K 31/573

(54) **OTIC COMPOSITIONS USEFUL FOR THE TREATMENT OF INFECTIONS OF THE INTERNAL AND EXTERNAL EAR IN MAMMALS**

(30) Priority: 27.02.2009 US 380463
(62) Divisional of application: 10746775.5
(71) Applicant: Foresight Biotherapeutics, Inc., New York, NY 10019 (US)
(72) Inventor: CAPRIOTTI, Joseph, A., New York, NY New York 10025 (US); LIANG, Bo, East Brunswick, NJ New Jersey 08816 (US); SAMSON, C., Michael, New York, NY New York 10016 (US); STEIN, Jason, New York, NY New York 10162 (US); WEISER, Michael, New York, NY New York 10012 (US)
(74) Representative: Carpmaels & Ransford LLP

(57) **Abstract**

Disclosed herein compositions including povidone-iodine (PVP-I) useful in the treatment of acute and chronic bacterial, viral and fungal infections of the internal, middle and external ear of mammals, including humans.

## Description

### BACKGROUND

Otitis externa (external ear infection) is an inflammation of the outer ear and ear canal. It is a common cause of earache in humans and a common problem in dogs, cats and other mammals. It also occurs in many other species. This disorder involves inflammation of the skin of the ear canal. The inflammation can be caused by active fungal, viral or bacterial organisms. The ear canal skin often swells and may become painful and tender to touch.

Otitis media (middle ear infection) occurs in the area between the ear drum and the inner ear, including the Eustachian tube. Otitis media is very common in childhood, with the average toddler experiencing two to three episodes a year, almost always accompanied by a viral upper respiratory infection (URI), mostly the common cold. The rhinoviruses and adenoviruses that cause many common cold symptoms cause swelling and congestion in the inner ear which can permanently damage middle ear structures. Otitis media is also frequently caused by a variety of bacteria and other viruses.

Furthermore, ear infection (particularly in children) is one of the many diseases that have become hard to treat with traditional antibiotic drugs because of antibiotic resistant bacteria and antibiotic-resistant microorganisms. Most cases of otitis media, for example, are caused by one of several major pathogens, Streptococcus pneumonia, Haemophilus influenza, Moraxella catarrhalia, Staphylococcus aureus, Staphylococcus epidermidis, or Pseudomonas aeruginosa. There is thus an urgent need to develop new, non-antibiotic approaches to prevent and manage these diseases.

### BRIEF SUMMARY OF THE INVENTION

The invention includes a method of treating a mammal having an otic infection, the method comprising contacting the ear of the mammal with a composition comprising povidone iodine (PVP-I) at a concentration of 0.01 %-5.0% and a steroid at a concentration of 0.01%-2.0%.

In an aspect, the otic condition is at least one member selected from the group consisting of bacterial otitis externa, malignant otitis, fungal otitis externa, otomycosis, otitis media, and otitis interna. In an aspect, the PVP-I is present at a concentration of 1.0%-3.0%. In another aspect, the PVP-I is present at a concentration of 2.0%.

In an aspect, the steroid is selected from the group consisting of a dexamethasone, a fluromethalone, a lotoprendol, a medrysone, a prednisolone, a difluprednate, a rimexolone, and a hydrocortisone. In another aspect, the steroid is dexamethasone or a salt thereof. In an aspect, the steroid is present at a concentration of 0.05%-0.1%. In another aspect, the steroid is present at a concentration of 0.1%.

In an aspect, the composition is contacted to the ear in the form of an ear drop, a zinc acetate composition, or an acetic acid composition.

### DETAILED DESCRIPTION OF THE INVENTION

For the treatment of otic infections, there are currently no effective antifungals, no effective antivirals and only one antibacterial available combined in the same dose form with anti-inflammatories. It is well known that more dilute concentrations of PVP-I exhibit a more potent antimicrobial effect in vitro, however previous attempts to produce a clinically effective dilute PVP-I otic solution have been so far unsuccessful. It is further noted that the formulation of PVP-I with other active ingredients can be complicated by reactivity of iodine species with other labile chemical moieties. Such reactive species are common on many steroids and non-steroidal anti-inflammatories.

### Definitions

As used herein, "ear" refers to the biological structures responsible for hearing and equilibrium in vertebrates, among other things. "Ear" also include the visible portions of the biological structures, such as those often present on mammals.

The term "otic" refers to the ear, in general.

The term "treating", as used herein, refers to a detectable improvement in an adverse condition and/or a lessening the symptoms of the condition upon contacting a mammal with an oral composition of the invention and/or according to a method of the invention. The term "treating" encompasses both a partial improvement in an adverse condition and a complete eradication (i.e., "cure") of the condition. In an aspect, an infection is treated. In another aspect, inflammation is treated. In another aspect, infection and inflammation are both treated.

### Treatment of Otic Infections

Cleaning in and around the ear canal, as well as treating otic infections, can sometimes be complicated by a buildup of cerumen, dead skin and other organic matter in and around the ear canal. Iodine is known to react with such substances, as iodine is chemically reactive, and active as a reducing agent. It is known, for example, that such substances in and around the ear canal can deplete the concentration of iodine in a 10% iodine solution that is used for such cleaning purposes, thereby depleting the cleaning and antimicrobial effectiveness of the iodine solution.

As disclosed herein, it has been surprisingly found that compositions comprising povidone-iodine and a steroid are advantageously effective as antimicrobial agents for otic indications. Additionally, it has been found that the inventive compositions are effective at lower concentrations of iodine than shown and/or used in the prior art.

It has now been determined that the compositions and formulations disclosed herein are surprisingly tolerable in the human ear. It is also disclosed herein that formulations of the invention have *in vitro* activity against many common bacterial, viral and fungal pathogens. In an aspect, the invention encompasses treatment of a mammalian ear. In an aspect, the mammal is a human.

In an embodiment, a condition treatable with a composition of the invention includes, but is not limited to, bacterial otitis externa, malignant otitis, fungal otitis externa, otomycosis, otitis media, and otitis interna.

### Antimicrobials - Povidone Iodine

In one embodiment, it is desirable to treat otic disorders as disclosed herein with both anti-inflammatories and antimicrobials suitable for the casuative organisms. Povidone iodine is an antimicrobial useful in the present invention.

Povidone-iodine (PVP-I) is a water-soluble complex of iodine with polyvinylpyrrolidone (PVP), with from 9.0% to 12.0% available iodine, calculated on a dry basis. PVP-I can be further formulated into topical antiseptic products as a solution (with surfactants and/or alcohol), aerosol or ointment at concentrations from 7.5% to 10%. These products are sold over-the-counter (OTC) and used in hospitals for cleansing and disinfecting the skin, preparing the skin preoperatively and treating infections susceptible to iodine. It is believed that the membrane proteins in the cell architecture are oxidized and subsequently denatured by free iodine generated from PVP-I solutions. This then leads to the disruption of cellular boundaries and the free passage of iodine into the cell.

Concentrations of PVP-I up to 10.0% (w/w, aqueous) are known to be safe for use in the external ear and similarly safe if exposed to the inner ear through a ruptured tympanic membrane.

### PVP-I + Steroid Compositions

In one embodiment, a stable, tolerable formulation of PVP-I with steroids has been developed, as set forth in U.S. Patent Application Publication No. 2007/0219170, incorporated herein by reference in its entirety. PVP-I and steroids are prepared in aqueous solution with common pharmaceutical excipients that is surprisingly stable at room temperature and elevated temperature in glass bottles and high-density polyurethane (HDPE) plastic bottles.

The affinity of free iodine for reaction with --OH, --SH and --NH functional groups is well described in the literature and forms the basis for the anti-microbial activity of iodine-containing solutions (Rackur H. J. Hosp. Infect., 1985; 6: 13-23, and references therein). Dexamethasone (9-Fluoro-11βα, 17, 21-trihydroxy-16α-methylpregna-1, 4-diene-3, 20-dione) contains three such moieties (--OH) at the 11, 17 and 21 positions and two double bonds. One of skill in the art will understand that these hydroxyl groups would be prone to covalent substitution reactions by the free iodine generated in the solution equilibrium reaction described above for PVP-I, and the double bonds would be prone to electrophilic iodination reactions.

Non-limiting examples of suitable steroids include: Dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, and hydrocortisone acetate A steroid is used at a concentration of 0.01 %-2.0%, and in another embodiment, 0.05%-1.0% by weight of the final composition. In an aspect, the steroid concentration is 0.01%, 0.02%, 0.03%, 0.04%, 0.05%, 0.06%, 0.07%, 0.08%, 0.09%, 0.1%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.3%, 0.31%, 0.32%, 0.33%, 0.34%, 0.35%, 0.36%, 0.37%, 0.38%, 0.39%, 0.40%, 0.41%, 0.42%, 0.43%, 0.44%, 0.45%, 0.46%, 0.47%, 0.48%, 0.49%, or 0.5%.

In an embodiment, as set forth in U.S. Patent Application No. 11/636,293, incorporated herein by reference in its entirety as U.S. Patent Application Publication No. 2007/0219170, various solutions of PVP-I and dexamethasone remain stable for many months, when prepared according to the compositions disclosed therein. Based on the stability data disclosed, such compositions may be stable for years. The reaction of dexamethasone and PVP-I does not proceed to any appreciable extent at room temperature, in light or dark, or over time. After 8 weeks, the available iodine in the combination (0.3% PVP-I starting concentration) decreased by 20%.

Previously, for a dexamethasone/PVP-I composition, the available iodine of a 0.625% PVP-I solution was determined to be 91% at 25° C and 98% at 4° C after 5 weeks storage, respectively. (Iryo Yakugaku 2003, 29(1), 62-65). Dexamethasone/PVP-I compositions set forth herein showed demonstrated a stabilized dilute PVP-I solution. After 8 weeks at room temperature, the available iodine in solutions with 0.5% and 1% PVP-I were over 99%.

The invention therefore includes compositions comprising PVP-I in a range from 0.01% to 10% (weight/weight or weight/volume) in suitable drug delivery vehicles with or without a steroid or non-steroidal anti-inflammatory agents. In an embodiment, PVP-I is in the range of 1.0-5.0%, including any specific concentration within that range. In another embodiment, PVP-I is in the range from 1.5-4.0%, and in another embodiment, from 2.0-3.0%. In an embodiment, the PVP-I concentration is 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, or 10.0%. In an embodiment, a steroid is used at a pharmaceutically-acceptable concentration.

The compositions are useful in the treatment of active infections from bacterial, mycobacterial, viral, fungal, or amoeba causes, as well as treatment to prevent such infections in appropriate clinical settings. In an embodiment, the invention provides a PVP-I + steroid composition that is non-reactive and stable, i.e., the PVP-I and the steroid are compatible. In an aspect, the steroid is dexamethasone.

### PVP-I + Non-Steroidal Anti-Inflammatory Compounds

It was also shown that PVP-I reacted with Ketorolac (a non-steroidal anti-inflammatory) rapidly and that the Ketorolac was completely consumed and the available iodine in the PVP-I complex was reduced significantly depending on the ratio between Ketorolac and PVP-I. The combination of PVP-I and dexamethasone sodium phosphate also proved to be less stable, but not overly reactive (some dissociation of PVP-I complex to an unknown polymeric complex was observed in the UV spectra and the iodine concentration was reduced approximately 5% after 12 weeks. It was further observed that PVP-I reacts immediately with proparacaine and releases free iodine rapidly.

Suitable anti-inflammatories for use in conjunction with compositions and methods herein include, but are not limited to, ketotifen fumarate, diclofenac sodium, nepafenac, bromfenac, flurbiprofen sodium, suprofen, celecoxib, naproxen, rofecoxib, or lodoxamide tromethamine.

### Pharmaceutical Compositions and Formulations

In another aspect, the invention provides topical pharmaceutical compositions for use in treating and relieving the symptoms of ear, including, but not limited to, otitis interna, otitis media and otitis externa (both acute and chronic). In an embodiment, the compositions comprise PVP-I in an amount effective to reduce the growth of infection causing microbes and a pharmaceutically acceptable carrier therefor. In an embodiment, PVP-I is present in an otic composition in the range of 0.1%-10%. In an embodiment, the otic compositions may additionally comprise a steroid, such as, but are not limited to, dexamethasone.

In compositions for topical administration, the mixtures are preferably formulated as aqueous solutions at a pH of 3.5 to 6.5. Preferably, the pH is adjusted to between 4 and 5. This pH range may be achieved by the inclusion of suitable acids/bases in the composition.

In an aspect, a composition may comprise one or more of an excipient, an antimicrobial agent, a preservative, a cosolvent, a surfactant, a viscosity agent, and/or a bioadhesive agent, as set forth in detail elsewhere herein.

In an aspect, a pharmaceutical preparation is a partially-alcoholic preparation. As will be understood by the skilled artisan, inclusion of a percentage of alcohol in the preparation will aid in the solubility of the components, including the steroid and the PVP-I. The alcohol component will also serve as a dehydrating component for the surface to which the preparation is applied. Alcohols useful in the invention include methanol, ethanol, and isopropanol, among others.

### Lubricants

In an embodiment, a composition may comprise one or more lubricants including, but are not limited to, propylene glycol, glycerin, polyethylene glycol, dextran, blended polyvinyl alcohols, polyvinyl alcohol, polyethylene glycol, light mineral oil, hydroxypropyl methylcellulose, hypromellose, carbopol, carbomer 940 (polyacrylic acid), polyvinyl pyrrolidone, white petrolatum, soy lecithin, and sodium carboxyl methylcellulose, as well as other agents known to those skilled in the art, or any combination thereof. Typically, such lubricants are employed at a level of from 0.1% to 2% by weight. In an embodiment, the lubricants are 1.0% Propylene glycol, 0.3% glycerin, 2.7% blended polyvinyl alcohols, 1% polyvinyl alcohol, 1% polyethylene glycol, light mineral oil, 0.3% hydroxypropyl methylcellulose, 1.0% soy lecithin, 0.25% or 0.5% sodium carboxyl methylcellulose. In another embodiment, the total weight of a PVP-I, artificial-tear based lubricant is between 0.1% and 4.5%.

### Additional Antimicrobial Agents and Antibiotics

Suitable antibiotic /antimicrobial agents include, but are not limited to, fluoroquinolones (ciprofloxacin, levofloxacin, ofloxacin, moxifloxacin, gatifloxacin, and the like); Aminoglycosides (tobramycin, gentamicin, neomycin, and the like); Polymyxin B Combinations (polymyxin B/trimethoprim, Polysporin polymyxin B/bacitracin Neosporin polymyxin B/neomycin/ gramicidin, and the like) and other antibiotics (azithromycin, ilotycin, erythromycin, bacitracin, and the like).

### Topical Anesthetics

Suitable topical anesthetics for the compositions and methods of the invention include, but are not limited to, lidocaine, tetracaine or a derivative or combination thereof.

### Anti-allergic Components

Anti-allergic components include, but are not limited to, epinastine, emedastine difumarate azelastine hydrochloride, olopatadine hydrochloride, olopatadine, ketotifen fumarate, pemirolast potassium, nedocromil, lodoxamide, cromolyn and cromolyn salts, as well as zinc acetate.

### Preservatives

Preservative agents include benzalkonium chloride, thimerosal, chlorobutanol, methyl paraben, propyl paraben, phenylethyl alcohol, EDTA, sorbic acid, Onamer M, other agents known to those skilled in the art, or a combination thereof. Typically such preservatives are employed at a level of from 0.001% to 1.0% by weight of final composition.

### Co-Solvents

The compositions of the invention may contain one or more optional co-solvents. The solubility of the components of the present compositions may be enhanced by a surfactant or other appropriate co-solvent in the composition. Such cosolvents/surfactants include polysorbate 20, 60, and 80, polyoxyethylene/polyoxypropylene surfactants (e.g. Pluronic F-68, F-84 and P-103), cyclodextrin, tyloxapol, other agents known to those skilled in the art, or a combination thereof. Typically such co-solvents are employed at a level of from 0.01% to 2% by weight of the final composition.

### Soothing Agents

Furthermore, the compositions may comprise an effective amount of a chemical agent to provide a cooling sensation to relieve mild otic irritation, enhance comfort, provide a refreshing effect, and improved sensation, when the PVP-I solution is applied to the ear. Such an agent encompasses various chemicals and chemical classes, including, but are not limited to, cooling agents such as menthol, menthol derivatives including methone glycerin acetyl and menthyl esters, carboxamides, menthane glycerol ketals, alkyl substituted ureas, sulfonamides, terpene analogs, furanones, and phosphine oxides; or camphor, and borneol.

### Viscosity Agents

The compositions of the invention may contain an optional viscosity agent - that is, an agent that can increase viscosity. Viscosity increased above that of simple aqueous solutions may be desirable to increase otic absorption of the active compound, to decrease variability in dispensing the formulation, to decrease physical separation of components of a suspension or emulsion of the formulation and/or to otherwise improve the otic formulation. Such viscosity builder agents include as examples polyvinyl alcohol, polyvinylpyrrolidone, methyl cellulose, hydroxypropylmethylcellulose, hydroxyethylcellulose, carboxymethylcellulose, hydroxypropylcellulose, other agents known to those skilled in the art, or a combination thereof. Such agents are typically employed at a level of from 0.01% to 2% by weight of the final composition.

### Bioadhesive Agents

Bioadhesive agents can be used in the compositions to increase the retention time of the drug gradient over the biological substrates. The bioadhesive agents may include but are not limited to: polyvinylpyrrolidone (PVP), xanthan gum, locust bean gum, acacia gum, hydroxypropyl methylcellulose (HPMC), sodium alginate, pectin, gelatin, carbomer, polyvinylalcohol, gellan gum, tragacanth, acacia, and sodium carboxymethyl cellulose.

### Formulations and Evaluation of Effectiveness

In an embodiment, methods and compositions of the invention can reduce the progression of infectious otitis externa such that no additional progression is detected. Any method can be used to determine whether or not the severity of a symptom or the progression rate of otitis externa is reduced. For example, a human having otitis externa can be questioned regarding pain or discomfort before and after treatment to determine whether a symptom of otitis externa (e.g., ear pain or ear itching) is reduced. In some cases, a mammal can be observed or tested for the severity of a symptom of otitis externa (e.g., ear discharge, sensitivity of the ear to pressure, sensitivity of the earlobe to touch, or reduced hearing) before and after treatment with an anti-infective compound (e.g., PVP-I) and a steroid to determine whether or not the severity of a symptom is reduced. In some cases, an otolaryngologist can assess the severity of otitis externa (e.g., by performing a physical examination and assigning a Grade 1 to 4 score, the characteristics of which are known in the art) before and after treatment to determine whether the severity of a symptom is reduced. To determine whether or not progression of otitis externa is reduced, a physical examination can be performed at different time points to determine the amount of erythema and/or edema in and around the ear canal. The amount of erythema and edema observed at different time points can be compared to assess the progression rate. After treatment as described herein, the progression rate can be determined again over another time interval to determine whether or not the progression rate has decreased.

Therefore, it will be understood that an effective amount of a composition comprising PVP-I and a steroid is any amount that reduces the severity of a symptom or the progression of otitis externa without producing significant toxicity to the mammal. For example, an effective amount of PVP-I can be from about 0.1% to about 10% (e.g., about 2%) povidone- iodine in an otic drop formulation. In an embodiment, an effective amount of a steroid such as dexamethasone can be from about 0.05% to about 1.0% (e.g., about 0.1%) dexamethasone in an otic drop formulation. In an embodiment, an effective amount of a composition comprising PVP-I and dexamethasone can be from about 2 drops to about 8 drops of an otic drop formulation containing about 2% povidone-iodine and about 0.10% dexamethasone applied to the ear.

In an aspect, an otic composition is a zinc acetate composition. In another aspect, an otic composition is an acetic acid composition.

If a mammal does not appear respond to a particular amount of a composition of the invention, then the amount of one or more of the PVP-I and the dexamethasone, for example, can be increased. After receiving this higher concentration, the mammal can be monitored for both responsiveness to the treatment and toxicity symptoms, and adjustments made accordingly. The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, immunocompetency of the mammal, and severity of the otitis externa may require an increase or decrease in the actual effective amount administered. The frequency of administration can be any frequency that reduces the severity of a symptom or progression rate of otitis externa without producing significant toxicity to the mammal. For example, the frequency of administration can be from about once daily to about four times daily (e.g., about twice daily). The frequency of administration can remain constant or can be variable during the duration of treatment.

In another aspect, a course of treatment with an anti-infective compound and a steroid can include rest periods. For example, an anti-infective and a steroid can be administered over a two week period followed by a two week rest period, and such a regimen can be repeated multiple times. As with the effective amount, various factors can influence the actual frequency of administration used for a particular application. For example, the effective amount, duration of treatment, use of multiple treatment agents, route of administration, immunocompetency of the mammal, and severity of the otitis externa may require an increase or decrease in administration frequency. An effective duration for administering a composition provided herein can be any duration that reduces the severity of a symptom or the progression rate of otitis externa without producing significant toxicity to the mammal. Thus, the effective duration can vary from several days to several weeks, months, or years. In general, the effective duration for the treatment of otitis externa can range in duration from several days to several weeks. In some cases, an effective duration can be for as long as an individual mammal is alive. Multiple factors can influence the actual effective duration used for a particular treatment. For example, an effective duration can vary with the frequency of administration, effective amount, use of multiple treatment agents, route of administration, immunocompetency of the mammal, and severity of the otitis externa.

The above diagnosis and treatment considerations can be applied in a similar manner to the treatment of otitis media and otitis interna.

### EXPERIMENTAL EXAMPLES

### Example 1:

In an embodiment, an otic composition is as follows:
1. 0.1% - 10.0% (w/w) polyvinylpyrrolidinone-iodine complex (Povidone Iodine), [1-Vinyl-2-pyrrolidinone polymers, iodine complex], USP, CAS 2565541-8
2.0.05 - 0.1% (w/w) dexamethasone [9-fluoro-11β,17,21-trihydroxy-16α-methylpregna-1,4-diene-3,20-dione], Micronized, USP, CAS 50-02-2
3. Excipients, as required for intended application
4. pH = 4.0 adjusted by addition of 0.1 N sodium hydroxide solution or sulfuric acid
5. Sterile water, USP, q.s. to 100%

### Example 2:

In an embodiment, an otic composition is as follows:
1. 2.0 % (w/w) polyvinylpyrrolidinone-iodine complex (Povidone Iodine), [1-Vinyl-2-pyrrolidinone polymers, iodine complex], USP, CAS 2565541-8
2. 0.1% (w/w) dexamethasone [9-fluoro-11β,17,21-trihydroxy-16α-methylpregna-1,4-diene-3,20-dione], Micronized, USP, CAS 50-02-2
3. 0.01% (w/w) edetate disodium dihydrate, USP, CAS 6381-92-6
4. 0.35% (w/w) sodium chloride salt, powder, USP, CAS 7647-14-5
5. 0.05% (w/w) tyloxapol [4-(1,1,3,3-Tetramethylbutyl)phenol Polymer with Formaldehyde and Oxirane] CAS 25301-02-4
6. 1.2% (w/w) sodium sulfate, anhydrous, USP, EP, BP, CAS 7757-82-6
7. 0.25% (w/w) hydroxyethylcellulose, 2000 cps NF, CAS 9004-62-0
8. pH = 4.0 adjusted by addition of 0.1 N sodium hydroxide solution or sulfuric acid
9. Sterile water, USP, q.s. to 100%

### Example 3:

In an embodiment, and otic formulation is prepared as follows:
Compounding process:
1. Weigh out all powders and record weights
2. Add 40% of sterile water for injection to an appropriate size beaker.
3. With the aid of a homogenizer add the Dexamethasone and Tyloxapol.
4. Pour the above solution into a 100ml serum vial with magnetic spin bar.
5. Use another 5% of water to rinse the beaker into the serum vial.
6. While spinning add Hydroxyethylcellulose, continue to spin until uniform.
7. Adjust pH of the above composition to 4.0.
8. QS to 50 ml.
9. Crimp and Autoclave the container holding the above.
10. After autoclave cycle completely spin the contents until cool.
11. In a separate beaker add 40% of sterile water for injection
12. Add the following ingredients one by one in the following order making sure each ingredient is completely dissolved before adding the next: NaCl, EDTA, Sodium Sulfate, Povidone Iodine
13. Adjust pH to 4.0.
14. QS to 50 ml.
15. Filter to sterilize the above.
16. Inject the filtered solution into the Autoclaved solution and let spin until uniform.
17. Dispense in amber glass bottles.

The invention has been described herein by reference to certain preferred embodiments. However, as obvious variations thereon will become apparent to those skilled in the art, the invention is not to be considered as limited thereto. All patents, patent applications, and references cited anywhere is hereby incorporated by reference in their entirety.
A non-exhaustive list of embodiments of the disclosure is set out in the following numbered aspects.
1. A method of treating a mammal having an otic infection, the method comprising contacting the ear of the mammal with a composition comprising:
   a. povidone iodine (PVP-I) at a concentration of 0.01%-5.0%; and
   b. a steroid at a concentration of 0.01%-2.0%.
2. The method of aspect 1, wherein the otic condition is at least one member selected from the group consisting of bacterial otitis externa, malignant otitis, fungal otitis externa, otomycosis, otitis media, and otitis interna.
3. The method of aspect 1, wherein the steroid is selected from the group consisting of a dexamethasone, a fluromethalone, a lotoprendol, a medrysone, a prednisolone, a difluprednate, a rimexolone, and a hydrocortisone.
4. The method of aspect 1, wherein the steroid is dexamethasone or a salt thereof.
5. The method of aspect 1, wherein the composition is contacted to the ear in the form of an ear drop or a zinc acetate composition.
6. The method of aspect 1, wherein the PVP-I is present at a concentration of 1.0%-3.0%.
7. The method of aspect 1, wherein the PVP-I is present at a concentration of 2.0%.
8. The method of aspect 1, wherein the steroid is present at a concentration of 0.05%-0.1%.
9. The method of aspect 1, wherein the steroid is present at a concentration of 0.1%.

## Claims

1. A composition comprising povidone iodine (PVP-I) and a steroid for use in a method of treating a mammal having an otic infection, wherein the method comprises contacting the ear of the mammal with the composition and wherein the composition comprises:
a. povidone iodine (PVP-I) at a concentration of 0.01%-5.0%; and
b. a steroid at a concentration of 0.01%-2.0%.

2. The use of a composition comprising povidone iodine (PVP-I) and a steroid for the manufacture of a medicament for the treatment of a mammal having an otic infection, wherein said composition comprises:
a. povidone iodine (PVP-I) at a concentration of 0.01%-5.0%; and
b. a steroid at a concentration of 0.01%-2.0%.

3. The composition for use as claimed in claim 1 or the use as claimed in claim 2, wherein
the composition comprises povidone iodine (PVP-I) at a concentration of 0.01%-1.0% weight/weight and a steroid at a concentration of 0.01%-2.0% weight/weight,
the steroid is dexamethasone or a salt thereof,
the otic infection is selected from the group consisting of bacterium otitis externa, malignant otitis, fungal otitis externa, otomycosis, otitis media, and otitis interna, and
after a period of 8 weeks at room temperature after combining PVP-I and dexamethasone to form the composition, the available iodine in solution is greater than 99%.

4. The composition for use as claimed in claim 1 or the use as claimed in claim 2, wherein the otic condition is at least one member selected from the group consisting of bacterium otitis externa, malignant otitis, fungal otitis externa, otomycosis, otitis media, and otitis interna.

5. The composition for use as claimed in claim 1 or the use as claimed in claim 2, wherein the steroid is selected from the group consisting of a dexamethasone, a fluromethalone, a lotoprendol, a medrysone, a prednisolone, a difluprednate, a rimexolone, and a hydrocortisone.

6. The composition for use as claimed in claim 1 or the use as claimed in claim 2, wherein the steroid is dexamethasone or a salt thereof.

7. The composition for use as claimed in claim 1 or the use as claimed in claim 2, wherein the composition is contacted to the ear in the form of an ear drop or a zinc acetate composition.

8. The composition for use as claimed in claim 1 or the use as claimed in claim 2, wherein the PVP-I is present at a concentration of 1.0%-3.0%.

9. The composition for use as claimed in claim 1 or the use as claimed in claim 2, wherein the PVP-I is present at a concentration of 2.0%.

10. The composition for use as claimed in claim 1 or the use as claimed in claim 2, wherein the steroid is present at a concentration of 0.05%-0.1%.

11. The composition for use as claimed in claim 1 or the use as claimed in claim 2, wherein the steroid is present at a concentration of 0.1%.

12. The composition for use as claimed in claim 3 or the use as claimed in claim 3, wherein the PVP-I is present at a concentration of 0.1-1.0% weight/weight.

13. The composition for use as claimed in claim 3 or the use as claimed in claim 3, wherein the PVP-I is present at a concentration of 1.0% weight/weight.

14. The composition for use as claimed in claim 3 or the use as claimed in claim 3, wherein the steroid is present at a concentration of 0.05%-0.1% weight/weight.

15. The composition for use as claimed in claim 3 or the use as claimed in claim 3, wherein the steroid is present at a concentration of 0.1% weight/weight.
